# EUROPÄISCHE PATENTSCHRIFT

(11) **EP 1 797 915 B1**
(45) Veröffentlichungstag und Bekanntmachung des Hinweises auf die Patenterteilung: **05.11.2008**
(21) Anmeldenummer: 06023801.1
(22) Anmeldetag: 16.11.2006
(51) Int. Cl.: A61M 1/16

(54) **Hämodialyse-Salzbehälter mit Belüftung**
Salt container with air vent for hemodialysis
Récipient de sel avec aération pour l'hémodialyse

(30) Priorität: 15.12.2005 DE 102005060290
(43) Veröffentlichungstag der Anmeldung: 20.06.2007
(73) Patentinhaber: Ritter GmbH, 86830 Schwabmünchen (DE)
(72) Erfinder: Ritter, Ralf, 86836 Untermeitingen (DE)
(74) Vertreter: Gallo, Wolfgang

(56) Entgegenhaltungen:
- EP-A- 1 627 650
- EP-A2- 0 278 100
- WO-A-83/01381
- FR-A1- 2 647 349
- US-A1- 4 056 100
- US-A1- 2002 134 721

## Beschreibung

Die Erfindung betrifft einen Salzbehälter aus Kunststoff für die Hämodialyse.

Dabei handelt es sich um Einwegbehälter, welche die für einen Hämodialysevorgang benötigte Bicarbonatsalzmenge enthalten, und die mittels einer Kupplungsverbindung zum Gebrauch an ein Hämodialysegerät anschließbar sind. Das Hämodialysegerät spült dann Dialysierflüssigkeit durch den Salzbehälter, um das Salz aufzunehmen.

Solche Salzbehälter sind sowohl in Gestalt flexibler Beutel (z.B. WO 99/06 083 A1, DE 696 16 073 T2) als auch in Form starrer Behälter (z.B. EP 0 112 295 A2) bekannt.

Die Patentschrift FR 2 647 349 offenbart einen Dialysatsalzbehälter mit einer Ventilöffnung, die mit einer Membran abgedichtet ist.

Die Patentschrift WO 83/01381 A1 offenbart eine Saugflasche für einen Säugling, die an ihrer Unterseite ein Belüftungsöffnung aufweist, welche mit einer Gummifolie verdeckt ist. Die Gummifolie löst sich von der Öffnung, sobald Unterdruck entsteht und erlaubt damit, dass Luft in die Saugflasche einströmt.

Am Ende des Vorgangs saugt das Dialysegerät die Dialysierflüssigkeit aus dem Salzbehälter ab. Bei den Salzbehältern in Gestalt flexibler Beutel wird aufgrund des im Beutelinneren erzeugten Unterdrucks der Beutel zusammengedrückt, so daß er sich problemlos entleert. Bei starren Salzbehältern, die gewisse Vorteile gegenüber flexiblen Beuteln haben, ist dieses Entleeren allerdings poblematisch, weil der starre Behälter nicht kollabieren kann. Dies führt dazu, daß die Flüssigkeit nur teilweise abgesaugt werden kann.

Der Erfindung liegt die Aufgabe zugrunde, einen steifen Salzbehälter für die Hämodialyse so auszubilden, daß ein problemloses Entleeren des Salzbehälters durch den vom Dialysegerät erzeugten Unterdruck möglich ist.

Diese Aufgabe wird gemäß der Erfindung durch die im Anspruch 1 angegebene Anordnung gelöst. Vorteilhafte Ausgestaltungen der Erfindung sind Gegenstand der Unteransprüche.

Erfindungsgemäß ist der steife Salzbehälter mit einem Belüftungsventil versehen, das unter besonderer Berücksichtigung des Salzbehälters als Einwegbehälter ausgebildet ist, und zwar so, daß es äußerst kostengünstig herstellbar ist und keinen Störkörper innerhalb des Behälters darstellt.

Die Erfindung wird nachstehend anhand eines Ausführungsbeispiels näher erläutert, das in den anliegenden Zeichnungen dargestellt ist. In den Zeichnungen zeigt:
- Fig. 1: eine Draufsicht auf einen steifen Salzbehälter nach der Erfindung ohne Deckel,
- Fig. 2: einen Längsschnitt durch den Behälter bei geschlossenem Siegel,
- Fig. 3: eine vergrößerte Darstellung des Bereichs des geschlossenen Siegels in Fig. 2,
- Fig. 4: einen Längsschnitt durch den Salzbehälter ähnlich Fig. 2 bei sich öffnendem Siegel,
- Fig. 5: eine vergrößerte Darstellung des Bereichs des sich öffnenden Siegels in Fig. 4,
- Fig. 6: eine Darstellung ähnlich Fig. 1 einer abgewandelten Ausführungsform,
- Fig. 7: eine vergrößerte Darstellung ähnlich Fig. 3 der Abwandlung nach Fig. 6,
- Fig. 8: eine Darstellung ähnlich Fig. 1 einer weiteren abgewandelten Ausführungsform, und
- Fig. 9: eine vergrößerte Darstellung ähnlich Fig. 3 der abgewandelten Ausführungsform nach Fig. 8.

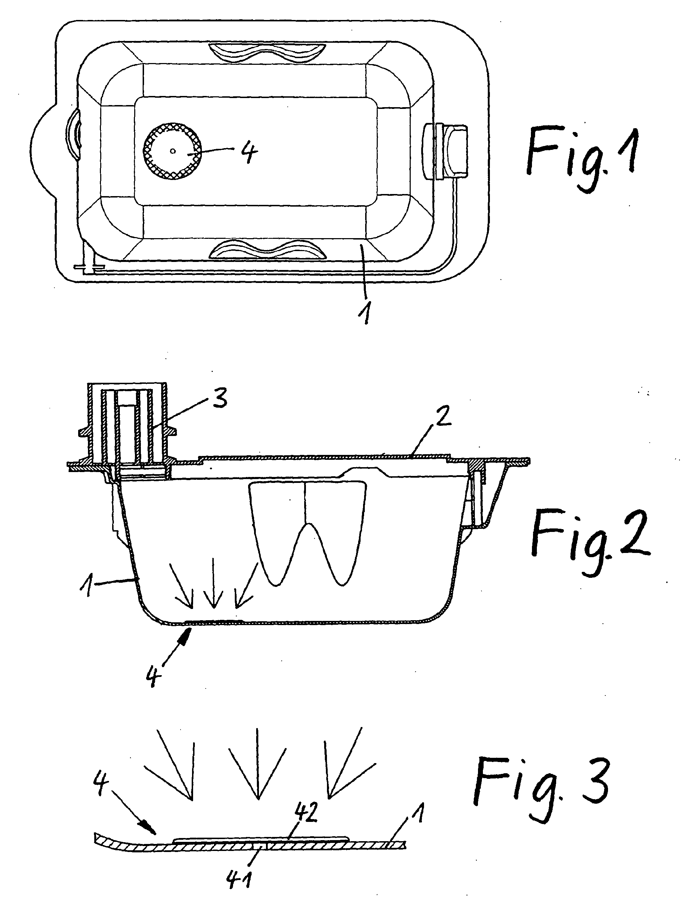

Die Figuren 1 und 2 zeigen einen steifen Salzbehälter, bestehend aus einem wannenförmigen Unterteil 1, einem Deckel 2, und einer am Deckel angeformten Koaxialsteckkupplung 3. Alle diese Teile sind in Figur 2 in der Schnittdarstellung sichtbar; bei der Draufsicht nach Figur 1 ist nur das Unterteil 1 ohne Deckel dargestellt. Die Salzfüllung ist nicht dargestellt. Im gefüllten gebrauchsfertigen Zustand des Salzbehälters sind Unterteil 1 und Deckel 2 miteinander verschweißt.

Die Ausbildung von Unterteil, Deckel und Koaxialsteckkupplung sind an sich bekannt und werden daher nicht im einzelnen beschrieben.

Das Unterteil 1 ist mit einem an seiner Bodenwand angeordneten Belüftungsventil 4 ausgestattet, um beim Absaugen der Dialysierflüssigkeit aus dem Behälter nach dem Ausspülen der Salzfüllung aus dem an ein Dialysegerät angeschlossenen Salzbehälter das vollständige Entleeren des Salzbehälters zu ermöglichen, in dem bei einwirkendem Unterdruck ein Eintritt von Außenluft ermöglicht wird.

Wesentlich ist bei diesem Belüftungsventil für den als Einwegbehälter konzipierten Salzbehälter, daß es einerseits mit geringem Aufwand herstellbar ist, andererseits aber zuverlässig funktioniert.

Wie aus der vergrößerten Darstellung nach Figur 3 ersichtlich ist, besteht das Belüftungsventil 4 aus einer in der Bodenwand des Unterteils 1 gebildeten Öffnung 41 und einem an der Innenseite der Bodenwand angebrachten, die Öffnung 41 verschließenden Siegel 42 aus einem Folienstück, das, wie Figur 1 erkennen läßt, kranzartig um die Öffnung 41 herum durch Kleben oder Ultraschallverschweißen mit der Bodenwand verbunden ist. In Figur 1 ist der kranzförmige Klebeflächenbereich durch Kreuzschraffur kenntlich gemacht.

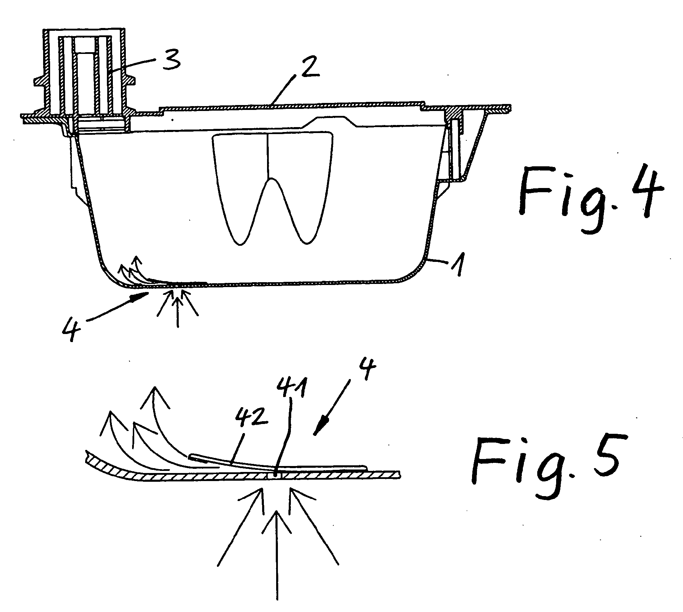

Im Betrieb des Salzbehälters an einem Dialysegerät wird beim Durchspülen des Salzbehälters mit Dialysierflüssigkeit zum Ausschwemmen des Bikarbonatsalzes das Siegel 42 durch den dann im Salzbehälter herrschenden Überdruck noch zusätzlich zu der Klebe- oder Schweißverbindung dichtend auf die Belüftungsöffnung 41 gepresst. Beim Absaugen der Dialysierflüssigkeit entsteht im Salzbehälter jedoch ein Unterdruck. Erreicht der Unterdruck im Salzbehälter einen bestimmten Grenzwert, löst sich das Siegel 42 unter der Einwirkung des äußeren Luftdrucks, der durch die Belüftungsöffnung 41 zutritt zur Siegelfolie hat, automatisch von der Bodenwand örtlich ab, wie in den Figuren 4 und 5 dargestellt ist, so daß Außenluft durch das Belüftungsventil 4 in den Behälter eintreten kann und die Flüssigkeit daher vollständig abgesaugt werden kann.

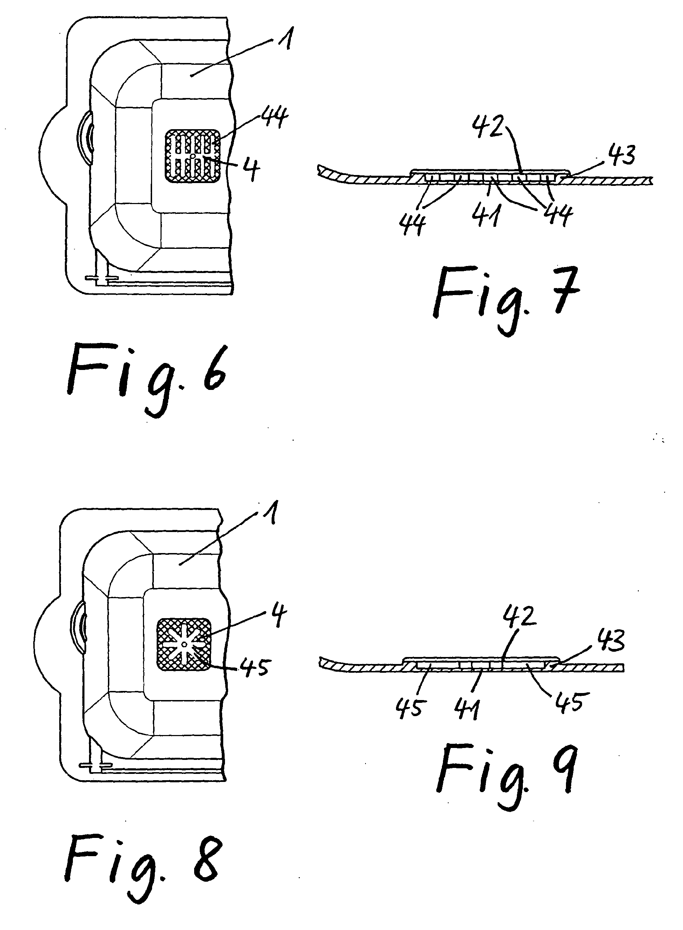

Die Figuren 6 und 7 sowie 8 und 9 zeigen jeweils mit Darstellungen ähnlich den Figuren 1 und 3 zwei abgewandelte Ausführungsbeispiele der Ausgestaltung des Belüftungsventils 4. Bei diesen beiden abgewandelten Ausführungsbeispielen ist an der Bodenwand des Unterteils 1 jeweils ein das Siegel 42 aufnehmender erhabener oder leicht verdickter Bodenwandbereich 43 vorgesehen. In diesen an der Innenwandseite der Bodenwand gebildeten erhabenen bzw. verdickten Bodenwandbereich 43 sind kanalartige Vertiefungen 44 (Figuren 6 und 7) bzw. 45 (Figuren 8 und 9) eingeformt, die bei der Ausführungsform nach den Figuren 6 und 7 die Gestalt einer Längsrinne mit mehreren diese kreuzenden Querrinnen haben und bei der Ausführungsform nach den Figuren 8 und 9 sternförmig angeordnet sind. Die um diese Vertiefungen stehen bleibenden erhabenen Bereiche sind in den Figuren 6 und 8 jeweils durch Kreuzschraffur dargestellt, und deren Oberflächen bilden die Klebe- bzw. Schweißflächenbereiche mit dem Siegel 42.

Während bei der Ausführungsform nach den Figuren 1 bis 5 die Verklebung bzw. Verschewißung des Siegels 42 mit der Bodenwand in einem örtlich definierten kreisförmigen Bereich mit einem gewissen Abstand um die Belüftungsöffnung 41 herum erfolgen muß, weil der Verklebungs- bzw. Verschweißungsbereich ja nur so groß sein darf, daß bei entsprechendem Unterdruck ein Aufreißen des Siegels an mindestens einer Stelle zuverlässig erfolgt und dann auch die Einwirkungsfläche auf das Siegel wesentlich größer sein muß als der Querschnitt der Belüftungsöffnung 41, haben die modifizierten Ausführungsformen nach den Figuren 6 und 7 sowie 8 und 9 den Vorteil, daß das Siegel 42 quasi vollflächig auf dem erhabenen Bodenwandbereich 43 aufgeklebt oder aufgeschweißt werden kann, also keine Maßnahmen zur Einhaltung eines bestimmten definierten Klebe- oder Schweißflächenbereichs getroffen zu werden brauchen. Denn durch die Vertiefungen 44 bzw. 45 werden dann die ausreichend große Außenluftdruckangriffsfläche auf die Siegelfolie bei Unterdruck im Salzbehälter und entsprechend schmale, ein örtliches Aufreißen des Siegels bei ausreichendem Unterdruck ermöglichenden Klebe- oder Schweißflächenbereiche erzeugt.

Die erfindungsgemäße Anordnung schafft ein Belüftungsventil, das bis zum Belüftungsfall zuverlässig dicht schließt, leicht herstellbar ist, keine beweglichen Teile hat, und auch nicht in den Behälterinnenraum vorsteht, so daß es weder durch Schütteln der Salzfüllung (z. B. bei Transport- und Handhabungsvorgängen) noch beim Durchspülen des Salzbehälters mit Dialysierflüssigkeit beeinträchtigt werden kann noch in irgend einer Weise das Füllen des Behälters mit Salz erschwert.

Für den Fachmann versteht es sich, daß das Belüftungsventil 4 an beliebiger Stelle der Behälterwand angeordnet sein kann, was statt am Unterteil 1 auch am Deckel 2 der Fall sein könnte. Dabei ist klar, daß man die Anordnungstelle in Abhängigkeit von der Orientierung des Salzbehälters in seiner Gebrauchslage nach dem Ankuppeln an ein Dialysegerät so wählt, daß das Belüftungsventil 4 in dieser Gebrauchslage des Salzbehälters möglichst weit oben liegt.

## Patentansprüche

1. Als Einwegbehälter ausgebildeter steifer Salzbehälter (1, 2) für die Hämodialyse, der mit einer zur Herstellung der Strömungsverbindungen mit einem Dialysegerät dienenden Kupplungsanordnung (3) versehen und im übrigen luftdicht verschlossen ist,
wobei der Salzbehälter ein an einer Behälterwand angeordnetes Belüftungsventil (4) mit einer in der Behälterwand gebildeten Belüftungsöffnung (41) und eine diese überdeckende, mit der Behälterwand durch örtliches Verkleben oder Verschweißen verbundenen Siegelfolie (42) umfaßt, **dadurch gekennzeichnet, daß** die Siegelfolie die Belüftungsöffnung an der Innenseite der Behälterwand überdeckt und daß die Verklebung oder Verschweißung so bemessen ist, daß bei Erreichen einer bestimmten Druckdifferenz zwischen dem Luftdruck außerhalb des Behälters und einem vom Dialysegerät erzeugten Unterdruck im Inneren des Behälters die Siegelfolie (41) mindestens örtlich aufreißt.

2. Salzbehälter nach Anspruch 1, wobei die Belüftungsöffnung (41) einen kleinen Querschnitt hat und der Verklebungs- oder Verschweißungsbereich der Siegelfolie (42) mit der Behälterwand einen deutlichen radialen Abstand von der Belüftungsöffnung (41) hat.

3. Salzbehälter nach Anspruch 1, wobei an der Innenseite der Behälterwand im Bereich der Belüftungsöffnung (41) Vertiefungen (44, 45) eingearbeitet sind die mit der Belüftungsöffnung (41) in Verbindung stehen, und die Verklebung oder Verschweißung der Siegelfolie (42) mit der Behälterwand auf den die Vertiefungen (44, 45) umgebenden Wandflächenbereichen erfolgt.

4. Salzbehälter nach Anspruch 3, wobei die Vertiefungen (44, 45) eine Anordnung von sternförmig oder in anderer geometrischer Anordnung um die Belüftungsöffnung (41) herum angeordneten flachen Kanälen gebildet sind.

5. Salzbehälter nach einem der Ansprüche 1 bis 4, wobei die Behälterwand in einem der Ausdehnung der Siegelfolie (42) entsprechenden Bereich mit einer vorzugsweise zur Wandinnenseite vorspringenden Wandverdickung ausgebildet ist.

## Claims

1. Rigid salt container (1, 2), realized as a disposable container, for haemodialysis, which is provided with a coupling arrangement (3) serving to effect the flow connections to a dialyser and which is otherwise closed in an airtight manner,
wherein the salt container comprises an air valve (4), which is arranged on a container wall and equipped with an air opening (41) formed in the container wall, and a sealing film (42), which covers this air opening and which is connected to the container wall through local bonding or welding, **characterized in that**
the sealing film covers the air opening on the inside of the container wall and **in that** the bonding or welding is so rated that, upon attainment of a defined pressure difference between the air pressure outside the container and a negative pressure, generated by the dialyser, inside the container, the sealing film (41) tears open, at least locally.

2. Salt container according to Claim 1, wherein the air opening (41) has a small cross-section and the bonding region or welding region of the sealing film (42) to the container wall has a distinct radial spacing from the air opening (41).

3. Salt container according to Claim 1, wherein recesses (44, 45) are sunk in on the inside of the container wall, in the region of the air opening (41), which recesses connect to the air opening (41), and the bonding or welding of the sealing film (42) to the container wall is effected on the wall surface regions surrounding the recesses (44, 45).

4. Salt container according to Claim 3, wherein the recesses (44, 45) are formed as an arrangement of flat channels arranged in a stellate manner or in another geometric arrangement around the air opening (41).

5. Salt container according to any one of Claims 1 to 4, wherein the container wall is realized in a region corresponding to the extent of the sealing film (42) and having a thickened wall region that preferably projects towards the inside of the wall.

## Revendications

1. Récipient de sel (1, 2) rigide conçu comme un récipient à usage unique pour l'hémodialyse qui est muni d'un dispositif d'accouplement (3) servant à établir les liaisons d'écoulement avec un appareil de dialyse, et est par ailleurs fermé de manière étanche à l'air,
le récipient de sel comprenant une soupape d'aération (4), disposée sur une paroi de récipient et munie d'un orifice d'aération (41) formé dans la paroi de récipient, et un film de scellement (42) recouvrant ledit orifice d'aération, relié à la paroi de récipient par collage ou soudage local, **caractérisé en ce que** le film de scellement recouvre l'orifice d'aération du côté intérieur de la paroi de récipient et que le collage ou le soudage est dimensionné de manière à ce que, lorsqu'une différence de pression déterminée entre la pression de l'air à l'extérieur du récipient et une dépression produite par l'appareil de dialyse à l'intérieur du récipient est atteinte, le film de scellement (41) s'arrache au moins localement.

2. Récipient de sel selon la revendication 1, dans lequel l'orifice d'aération (41) a une petite section transversale et la zone de collage ou de soudage du film de scellement (42) avec la paroi de récipient est à une distance radiale nette de l'orifice d'aération (41).

3. Récipient de sel selon la revendication 1, dans lequel des creux (44, 45) ménagés du côté intérieur de la paroi de récipient dans la zone de l'orifice d'aération (41) sont en liaison avec l'orifice d'aération (41), et le collage ou soudage du film de scellement (42) avec la paroi de récipient a lieu sur les zones de surface de paroi entourant les creux (44, 45).

4. Récipient de sel selon la revendication 3, dans lequel les creux (44, 45) sont formés par un agencement de canaux plats disposés en étoile ou dans une autre disposition géométrique tout autour de l'orifice d'aération (41).

5. Récipient de sel selon l'une des revendications 1 à 4, dans lequel la paroi de récipient est réalisée, dans une zone correspondant à l'étendue du film de scellement (42), avec un épaississement de paroi dépassant de préférence vers le côté intérieur de la paroi.
